# EUROPEAN PATENT APPLICATION

(11) **EP 3 323 454 A1**
(43) Date of publication of application: **23.05.2018**
(21) Application number: 16199019.7
(22) Date of filing: 16.11.2016
(51) Int. Cl.: A61M 11/00, A61M 15/00, B05B 17/00

(54) **PORTABLE MESH NEBULIZER**

(71) Applicant: Huang, Yu-Kang, Taichung City (TW)
(72) Inventor: Huang, Yu-Kang, Taichung City (TW)
(74) Representative: Horak, Michael

(57) **Abstract**

The portable mesh nebulizer (3) may be assembled from and dis-assembled into a number of functional members including a reservoir member (31), a spray member (32), and a vibration member (33). A variety of these functional members therefore may be put together to achieve different treatment effects, depending on user requirement and the liquid medication (a) to be applied. The present invention therefore not only is cost effective, but also is significantly convenient both in terms of storage and transportation.

## Description

### BACKGROUND OF THE INVENTION

### (a) Technical Field of the Invention

The present invention is generally related to nebulizers, and more particular to an easy-to-carry portable mesh nebulizer.

### (b) Description of the Prior Art

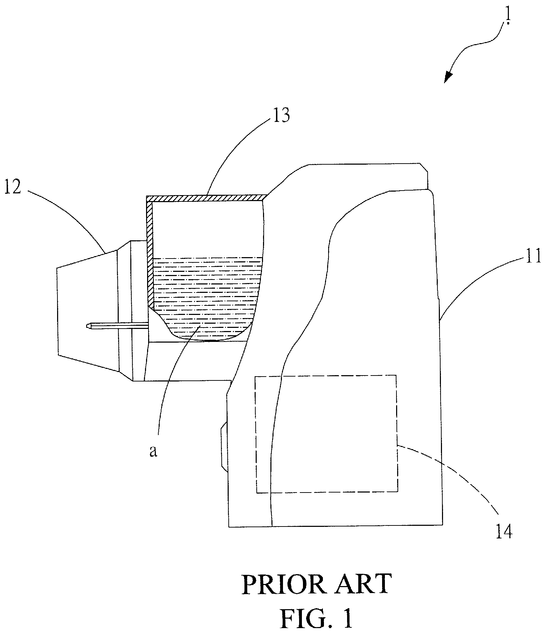

As shown in FIG 1, a conventional mesh nebulizer 1 includes a main member 11, a nozzle 12 located to a side of the main member 11, a reservoir member 13 storing medication liquid (a) configured on the main member 11 and connected to the nozzle 12, and a vibration member 14 inside the main member 11 connected to the reservoir member 13 (represented by the dotted box). The vibration by the vibration member 14 beneath the reservoir member 13 produces and releases a mist of droplets of the medication liquid (a) out of the nozzle 12.

The conventional nebulizer 1 is an integral device having a fixed amount of medication liquid (a) stored in the reservoir member 13 and only a single form of mist is available. In other words, the reservoir member 13 is not replaceable and the conventional nebulizer 1 cannot deliver different medication liquids (a) as needed. The nozzle 12 also cannot be replaced by one providing a different form of mist. Therefore the usage cost of a conventional nebulizer 1 cannot be reduced as a user may have to prepare multiple conventional nebulizers 1, one for each medication liquid (a) required. On the other hand, the reservoir member 13, the nozzle 12, and the vibration member 14 cannot be dismantled, thereby leading to inconvenience when carrying the conventional nebulizers 1.

### SUMMARY OF THE INVENTION

Therefore the objective of the present invention is to teach a portable mesh nebulizer capable of being assembled and dis-assembled quickly, providing great convenience to carry.

The portable mesh nebulizer includes a reservoir member, a spray member joined to a lateral side of the reservoir member, and a vibration member joined to a bottom side of the reservoir member. The reservoir member includes a cup element for storing a liquid medication, a first connection element on a lateral side of the cup element for joining with the spray member; a cap element sealing a top side of the cup element, and a second connection element on a bottom side of the cup element opposite to the cap element for joining with the vibration member. The vibration member includes a circuit element, an interface element on a lateral side of the vibration member, an electricity storage element respectively and electrically connecting the circuit element and the interface element, and a plug element on a top side of the vibration member joining to the second connection element. The portable mesh nebulizer may be assembled from and dis-assembled into a number of these functional members. Various functional members such as the reservoir member, spray member, and vibration member therefore may be put together to achieve different effects, depending on user requirement and the liquid medication to be applied. The present invention therefore not only is cost effective, but also is significantly convenient both in terms of storage and transportation.

The foregoing objectives and summary provide only a brief introduction to the present invention. To fully appreciate these and other objects of the present invention as well as the invention itself, all of which will become apparent to those skilled in the art, the following detailed description of the invention and the claims should be read in conjunction with the accompanying drawings. Throughout the specification and drawings identical reference numerals refer to identical or similar parts.

Many other advantages and features of the present invention will become manifest to those versed in the art upon making reference to the detailed description and the accompanying sheets of drawings in which a preferred structural embodiment incorporating the principles of the present invention is shown by way of illustrative example.

### BRIEF DESCRIPTION OF THE DRAWINGS

FIG 1 is a schematic diagram showing a conventional mesh nebulizer.
FIG 2 is a perspective breakdown diagram showing a portable meshi nebulizer according to an embodiment of the present invention.
FIG 3 is a schematic sectional diagram showing the portable mesh nebulizer of FIG 2.
FIG 4 is a perspective diagram showing an application scenario of the portable mesh nebulize of FIG 2.
FIG 5 is a perspective diagram showing another application scenario of the portable mesh nebulize of FIG 2.
FIG 6 is a perspective diagram showing yet another application scenario of the portable mesh nebulize of FIG 2.

### DETAILED DESCRIPTION OF THE PREFERRED EMBODIMENTS

The following descriptions are exemplary embodiments only, and are not intended to limit the scope, applicability or configuration of the invention in any way. Rather, the following description provides a convenient illustration for implementing exemplary embodiments of the invention. Various changes to the described embodiments may be made in the function and arrangement of the elements described without departing from the scope of the invention as set forth in the appended claims.

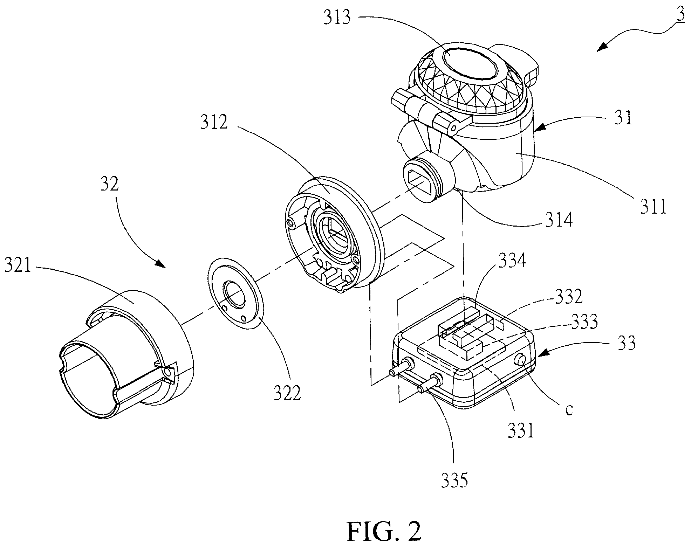

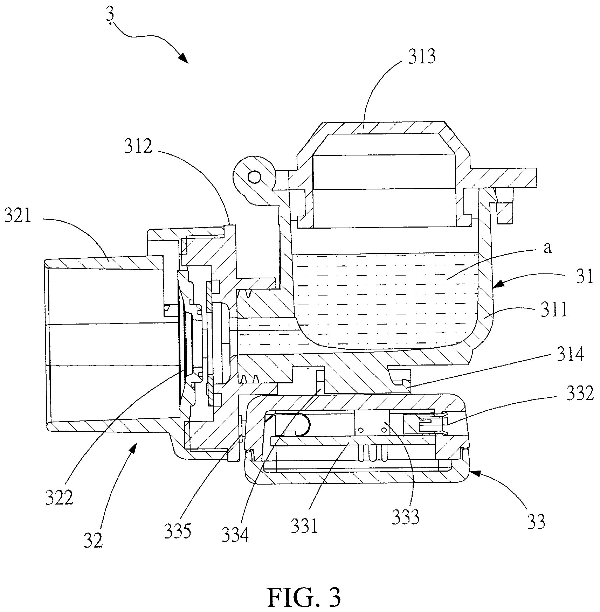

As shown in FIGS. 2 and 3, a portable mesh nebulizer 3 according to an embodiment of the present invention includes a reservoir member 31, a spray member 32 joined to a lateral side of the reservoir member 31, and a vibration member 33 joined to a bottom side of the reservoir member 31. The reservoir member 31 includes a cup element 311 for storing a liquid medication (a), a first connection element 312 on a lateral side of the cup element 311 for joining with the spray member 32, a cap element 313 sealing a top side of the cup element 311 for preventing the liquid medication (a) from leakage, and a second connection element 314 on a bottom side of the cup element 311 opposite to the cap element 313 for joining with the vibration member 33. The spray member 32 includes a nozzle element 321, and a mist forming element 322 between the nozzle element 321 and the first connection element 312 adjustable to produce mists of different forms. The nozzle element 311 can also be of different shapes and dimensions as demanded.

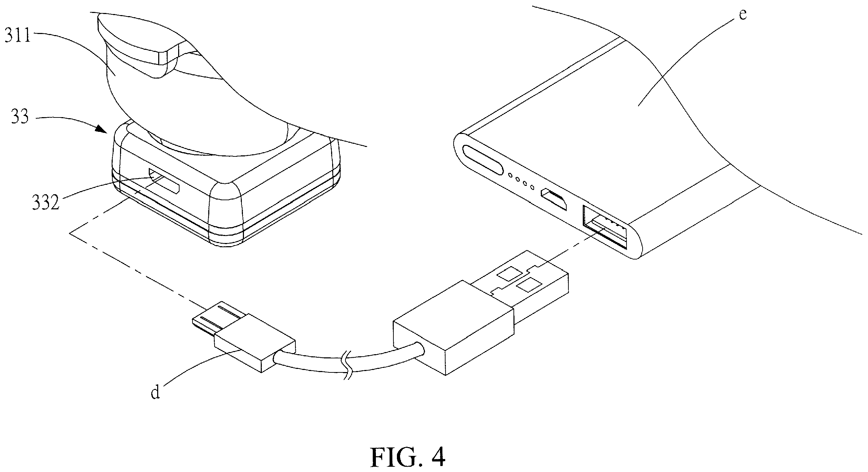

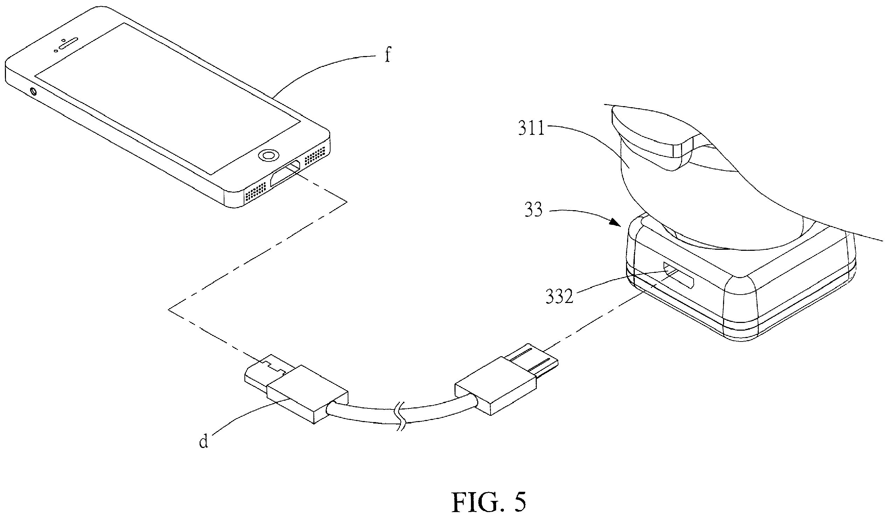

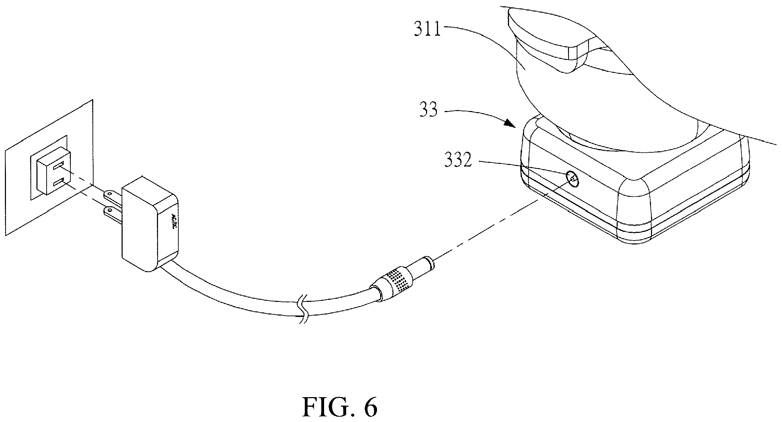

The vibration member 33 includes a circuit element 331, an interface element 332 on a lateral side of the vibration member 33, an electricity storage element 333 respectively and electrically connecting the circuit element 331 and the interface element 332, a plug element 334 on a top side of the vibration member 33 joining to the second connection element 314, and a number of terminals 335 on another lateral side of the vibration member 33 opposite to the interface element 332 connecting the circuit element 331. When the reservoir member 31 and the vibration member 33 are joined together by the second connection element 314 and the plug element 334, the terminals 335 are extended into the first connection element 312 and connected to the mist forming element 322. The interface element 332 may be a USB socket and, by a Micro/USB cable (d), connected to an external portable power (e) or a cellular phone (f) as shown in FIGS. 4 and 5. The electricity storage element 333 is as such charged by the portable power (e) or the cellular phone (f) through the interface element 332. The interface element 332 may also be a socket for a power adaptor connecting a wall mains as shown in FIG 6. The electricity storage element 333, denoted by the dotted box in FIG 2, may be a lithium battery capable of storing a large amount of the electricity and sustaining the vibration member 33 for an extended period of time. The circuit element 331 may provide three types of vibration modes: strong, medium, and weak so that a user may choose one vibration mode to fit the characteristic of the liquid medication (a). The circuit element 331's trigger for the vibration modes may be delivered to the mist forming element 322 through the terminals 335 as well so that the mist forming element 322 operates synchronously.

As shown in FIGS. 2 and 3, the interface element 332 is first connected to an external power source (not shown in FIGS. 2 and 3) so as to charge and store electricity in the electricity storage element 333. After the electricity storage element 333 is charged, the interface element 332 may be disconnected from the external power source. Then, a reservoir member 31 fitting the user's requirement is prepared and joined to the vibration member 33 by joining the second connection element 314 and the plug element 334. A switch (c) of the vibration member 33 is engaged to turn on the vibration member 33 and the circuit element 331 draws electricity from the electricity storage element 333 to operate. The circuit element 331 produces a mode of vibration delivered to the cup element 311 so that the liquid medication (a) in the cup element 311 is vibrated and nebulized. As the nebulized liquid medication (a) passes through the nozzle element 321, it is further processed by the mist forming element 322 between the nozzle element 321 and the first connection element 312 so that the nebulized liquid medication (a) is sprayed in a specific form of mist. The reservoir member 31, the spray member 32, and the vibration member 33 are detachable and therefore those fitting a specific need may be assembled together. Together with the various vibration modes of the vibration member 33, a superior treatment effect may be obtained from the liquid medication (a) in the cup element 311 than those conventional nebulizers having a fixedly attached reservoir member and a single mode of vibration. The present invention is more economically efficient and, as the reservoir member 31, the spray member 32, and the vibration member 33 may be dis-assembled, the storage and transportation of the portable mesh nebulizer 3 is significantly more convenient.

In summary, the portable mesh nebulizer of the present invention may be assembled from and dis-assembled into a number of functional members such as the reservoir member, spray member, and vibration member. Various functional members therefore may be put together to achieve different effects, depending on user requirement and the liquid medication to be applied. The present invention therefore not only is cost effective, but also is significantly convenient both in terms of storage and transportation.

While certain novel features of this invention have been shown and described and are pointed out in the annexed claim, it is not intended to be limited to the details above, since it will be understood that various omissions, modifications, substitutions and changes in the forms and details of the device illustrated and in its operation can be made by those skilled in the art without departing in any way from the claims of the present invention.

## Claims

1. A portable mesh nebulizer, comprising a reservoir member (31), a spray member (32) joined to a lateral side of the reservoir member (31), and a vibration member (33) joined to a bottom side of the reservoir member (31);
wherein the reservoir member (31) comprises a cup element (311) for storing a liquid medication (a), a first connection element (312) on a lateral side of the cup element (311) for joining with the spray member (32); a cap element (313) sealing a top side of the cup element (311), and a second connection element (314) on a bottom side of the cup element (311) opposite to the cap element (313) for joining with the vibration member (33);
the vibration member (33) comprises a circuit element (331), an interface element (332) on a lateral side of the vibration member (33), an electricity storage element (333) respectively and electrically connecting the circuit element (331) and the interface element (332), and a plug element (334) on a top side of the vibration member (33) joining to the second connection element (314);
the circuit element (331) produces a mode of vibration delivered to the cup element (311) so that the liquid medication (a) in the cup element (311) is vibrated and nebulized into a nebulized liquid medication; and
the spray member (32) sprays the nebulized liquid medication from the reservoir member (31) in a form of mist.

2. The portable mesh nebulizer according to claim 1, wherein the spray member (32) comprises a nozzle element (321) connecting the first connection element (312), and a mist forming element (322) between the nozzle element (321) and the first connection element (312) adjustable to produce mists of different forms.

3. The portable mesh nebulizer according to claim 1, wherein at least a terminal (335) is configured on another lateral side of the vibration member (33) opposite to the interface element (332) and connected to the circuit element (331); and the terminal (335) is extended into the first connection element (312) and connected to the mist forming element (322).

4. The portable mesh nebulizer according to claim 1, wherein the interface element (332) is a Universal Serial Bus (USB) socket.

5. The portable mesh nebulizer according to claim 1, wherein the interface element (332) is a socket for a power adaptor connecting to mains.
